# EUROPÄISCHE PATENTSCHRIFT

(11) **EP 2 594 937 B1**
(45) Veröffentlichungstag und Bekanntmachung des Hinweises auf die Patenterteilung: **22.07.2015**
(21) Anmeldenummer: 11189576.9
(22) Anmeldetag: 17.11.2011
(51) Int. Cl.: G01N 33/00, G01N 21/17

(54) **Gassensor zur Detektion von Wasserstoffgas**
Gas sensor for detection of hydrogen gas
Capteur de gaz pour la détection de gaz d'hydrogène

(43) Veröffentlichungstag der Anmeldung: 22.05.2013
(73) Patentinhaber: Justus-Liebig-Universität Gießen, 35390 Gießen (DE)
(72) Erfinder: Meyer, Prof. Bruno, 35440 Großen-Linden (DE); Polity, Angelika, 61231 Bad Nauheim (DE); Dietrich, Marc Konstantin, 35390 Giessen (DE); Laufer, Andreas, 35444 Biebertal (DE)
(74) Vertreter: Patentanwälte Olbricht Buchhold Keulertz

(56) Entgegenhaltungen:
- MARC K DIETRICH ET AL: "DS 42.105: Optical switching properties and durability of a Mg-Fe alloy based thin film hydrogen sensor", VERHANDLUNGEN DER DEUTSCHEN PHYSIKALISCHEN GESELLSCHAFT, 16. März 2011 (2011-03-16), XP55020122, Dresden
- BAO S ET AL: "Optical property and cycling durability of polytetrafluoroethylene top-covered and metal buffer layer inserted Mg-Ni switchable mirror", SOLAR ENERGY MATERIALS AND SOLAR CELLS, ELSEVIER SCIENCE PUBLISHERS, AMSTERDAM, NL, Bd. 93, Nr. 9, 1. September 2009 (2009-09-01), Seiten 1642-1646, XP026251048, ISSN: 0927-0248, DOI: 10.1016/J.SOLMAT.2009.05.002 [gefunden am 2009-05-23]
- GÜNTHER HAAS ET AL: "MM 26.50 Properties and stability of hydrogen sensors based on magnesium-iron thin films", VERHANDLUNGEN DER DEUTSCHEN PHYSIKALISCHEN GESELLSCHAFT, 23. März 2010 (2010-03-23), XP55020171,
- BAO ET AL: "Metal buffer layer inserted switchable mirrors", SOLAR ENERGY MATERIALS AND SOLAR CELLS, ELSEVIER SCIENCE PUBLISHERS, AMSTERDAM, NL, Bd. 92, Nr. 2, 14. November 2007 (2007-11-14), Seiten 216-223, XP022344351, ISSN: 0927-0248, DOI: 10.1016/J.SOLMAT.2007.02.023
- BAO ET AL: "The effect of polymer coatings on switching behavior and cycling durability of Pd/Mg-Ni thin films", APPLIED SURFACE SCIENCE, ELSEVIER, AMSTERDAM, NL, Bd. 253, Nr. 14, 19. April 2007 (2007-04-19), Seiten 6268-6272, XP022033106, ISSN: 0169-4332, DOI: 10.1016/J.APSUSC.2007.01.114
- YOSHIMURA K ET AL: "ROOM-TEMPERATURE HYDROGEN SENSOR BASED ON PD-CAPPED MG2NI THIN FILM", JAPANESE JOURNAL OF APPLIED PHYSICS, JAPAN SOCIETY OF APPLIED PHYSICS, JP, Bd. 43, Nr. 4B, 15. April 2004 (2004-04-15), Seiten L507-L509, XP001222348, ISSN: 0021-4922, DOI: 10.1143/JJAP.43.L507
- Anonymous: "Duden | Platinoid | Rechtschreibung, Bedeutung, Definition, Herkunft", , 2013, XP055126797, Retrieved from the Internet: URL:http://www.duden.de/rechtschreibung/Pl atinoid [retrieved on 2014-07-03]

## Beschreibung

Die Erfindung betrifft einen Gassensor zur Detektion von Wasserstoffgas gemäß dem Oberbegriff von Anspruch 1, die Verwendung eines solchen Gassensors in einem mobilen Wasserstoffdetektor nach Anspruch 8 und einen mobilen Wasserstoffdetektor gemäß Anspruch 10.

Gassensoren zur Detektion von Wasserstoffgas werden unter anderem verwendet, um die Sicherheit von Wasserstofftanks zu kontrollieren. Dabei muss sichergestellt werden, dass die untere Explosionsgrenze von 4% Wasserstoff in Luft im Falle eines Austritts von Wasserstoffgas aus einem Tank auf keinen Fall überschritten wird.

Im Stand der Technik sind eine Reihe solcher Sensoren bekannt, die zum Teil auf unterschiedlichen Messverfahren beruhen. So sind einerseits optische Sensoren bekannt, die eine Veränderung der Transmissivität feststellen, als auch elektrisch lesbare Sensoren, die eine Widerstandsänderung nachweisen. Eine wesentliche Anforderung an solche Wasserstoff-Sensoren ist, dass sie eine hohe Sensitivität, Selektivität und Stabilität aufweisen.

Yoshimura et al. (K. Yoshimura, Y. Yamada, M. Okada, M. Tazawa and P. Jin: "Room-Temperature Hydrogen Sensor Based on Pd-Capped Mg2Ni Thin Films", Japanese Journal of Applied Physics, L507-L509) beschreiben beispielsweise einen elektrisch lesbaren Wasserstoff-Sensor, der aus einem Glassubstrat, einer funktionellen Mg₂Ni-Schicht und einer Palladium-Schicht besteht. Die Palladium-Schicht dient dabei als Mittel zur Aktivierung oder Spaltung von Wasserstoff. Die Mg₂Ni-Schicht wird zum Nachweis des aktivierten bzw. gespaltenen Wasserstoffs verwendet.

Die Veröffentlichung von S. Bao, Y. Yamada, K. Tajima, M. Okada und K. Yoshimura in Solar Energy Materials & Solar Cells, 93, 2009, 1642-1646 ("Optical property and cycling durability of polytetrafluoroethylene top-covered and metal buffer layer inserted Mg-Ni switchable mirror") betrifft Schichten für schaltbare Spiegel, wobei eine schaltbare Dünnschicht aus seiner Mg/Ni-Legierung besteht. Diese schaltbaren Spiegel sind für Fenster-Anwendungen vorgesehen.

Die WO 2007/062629 A2 beschreibt einen Gassensor, der sowohl optisch als auch elektrisch auslesbar ist. Dieser Gassensor besteht aus einem Schichtsystem mit einer Substratschicht, einer funktionellen Schicht und einer Schicht zur Aktivierung oder Spaltung von Wasserstoff. Als Substratschicht wird ein transparentes Material wie z.B. Glas oder Kunststoff verwendet. Die Schicht zur Wasserstoffaktivierung bzw. -spaltung besteht aus Platin oder Palladium. Als funktionelle Schicht mit deren Hilfe der aktivierte bzw. gespaltene Wasserstoff nachweisbar ist, wird eine Legierung von Magnesium mit Nickel oder Aluminium verwendet. Die Magnesium-Aluminium-Legierungen können zusätzlich weitere Elemente aus der Gruppe Li, Be, Na, Ti, V, Cr, Mn, Fe, Co, Ni, Zr, Nb, Mo, Tc, Ru, Rh, Pd, Ag, Cd, Hf, Ta, W, Re, Os, Ir, Pt, Au, Hg, Si, K, Ca, Ga, Ge, As, Zn, Cu aufweisen.

Laufer (A. Laufer: "Neue Generation von Wasserstoffdetektoren basierend auf MagnesiumMetall-Verbindungen" Diplomarbeit an der Justus-Liebig-Universität Gießen 2007) offenbart die Verwendung von Mg-Fe-Legierungen als aktive Schicht in einem Sensor, der ebenfalls aus einer Substratschicht, einer funktionellen Schicht und einer Schicht zur Aktivierung von Wasserstoff besteht. Dieser Sensor ist für die Detektion von Wasserstoffgas-Konzentrationen < 1% in Luft geeignet. Sensorsysteme mit Mg-Fe-Legierungen weisen im Bereich von 1 -0,1% Wasserstoff die größte Signaländerung auf.

Die Posterpräsentation DS 42.105 von M. K. Dietrich, G. Haas, A. Portz, A. Laufer, A. Polity und B. K. Meyer ("Optical switching properties and durability of a Mg-Fe alloy based thin film hydrogen sensor") am 16.03.2011 während der Veranstaltung "Verhandlungen der deutschen physikalischen Gesellschaft" in Dresden betrifft einen auf Mg-Fe basierenden Dünnschicht-Wasserstoffsensor, wobei dieser aus einer Funktionsschicht von MgFe, einer Zwischenschicht ("buffer layer") aus Titan, einer aus Pd bestehenden Katalysatorschicht und einer Deckschicht aus PTFE aufgebaut ist.

Problematisch ist bei den oben genannten Lösungen häufig, dass die Ansprechzeiten der bisher bekannten Sensoren oft > 10 s betragen können. Darüber hinaus können die bekannten Wasserstoff-Sensoren ungünstige temperaturabhängige Funktionsbereiche aufweisen und einen hohen Energieverbrauch haben. Auch die mobile Einsatzfähigkeit ist nicht immer gegeben.

Aufgabe der vorliegenden Erfindung ist es daher, einen verbesserten Gassensor zu Detektion von Wasserstoffgas bereit zustellen, der diese und andere Nachteile im Stand der Technik überwindet. Insbesondere soll ein Gassensor bereitgestellt werden, der eine möglichst schnelle Ansprechzeit, einen weitgehend temperaturunabhängigen Funktionsbereich und geringen Energieverbrauch aufweist. Der Gassensor soll außerdem möglichst mobil einsetzbar, robust und im Verhältnis preiswert herstellbar sein.

Hauptmerkmale der Erfindung sind im kennzeichnenden Teil von Anspruch 1 angegeben. Ausgestaltungen sind Gegenstand der Ansprüche 2 bis 7 und 9, sowie der nebengeordneten Ansprüche 8 und 10.

Bei einem Gassensor zur Detektion von Wasserstoffgas, wobei der Gassensor wenigstens eine Substratschicht, eine funktionelle Schicht und eine Deckschicht aufweist, wobei die funktionelle Schicht zwischen der Substratschicht und der Deckschicht angeordnet ist, sieht die Erfindung vor, dass zwischen der funktionellen Schicht und der Deckschicht eine Katalysatorschicht und eine Zwischenschicht angeordnet sind und dass die funktionelle Schicht eine Mg/Ti-Schicht ist.

Besonders vorteilhaft sind bei einem so gestalteten Gassensor, die zwischen der funktionellen Mg/Ti-Schicht und der Deckschicht angeordneten Schichten. In der Katalysatorschicht kann sich der nachzuweisende Wasserstoff lösen. Das führt zur Dissoziation des Wasserstoffs. Der dissoziierte Wasserstoff kann aus der Katalysatorschicht in die Zwischenschicht übergehen und von dort in die funktionelle Schicht, die in dessen Gegenwart vom metallischen in den Hydridzustand übergeht. Die Zwischenschicht kann die funktionelle Schicht auf diese Weise vor Degradationseffekten schützen, die durch Volumenaufweitungen der Katalysatorschicht durch die Absorption des Wasserstoffs entstehen. Die funktionelle Schicht, die Zwischenschicht und die Katalysatorschicht können dabei derart zusammenwirken, dass einerseits eine sehr kurze Ansprechzeit auf das einwirkende Wasserstoffgas erreicht wird. Andererseits kann die funktionelle Schicht sehr schnell wieder vom Hydrid-Zustand in den metallischen Ausgangszustand zurück versetzt werden. Der erfindungsgemäße Sensor ist insofern von hoher Sensibilität und kann sehr selektiv Wasserstoff nachweisen. Gleichzeitig schützt die Deckschicht die empfindlichen darunter liegenden Schichten gegenüber nachteiligen Umwelteinflüssen.

Die zwischen der Katalysatorschicht und der funktionellen Schicht angeordnete Zwischenschicht besteht aus einem hydrierbaren Metall. Bei einem solchen Metall handelt es sich um ein Übergangsmetall, wie Ti, V, V/Ti, Nb, Ta, Pt und Fe. Besonders günstig ist dabei die Verwendung der gut handhabbaren und in ausreichenden Mengen erhältlichen Metalle Ti oder V, insbesondere Ti. Die Zwischenschicht kann dabei zusätzlich als Schutz vor Oxidation für die darunter liegende Mg/Ti- Schicht dienen. Dadurch kann die Standzeit des erfindungsgemäßen Gassensors deutlich verbessert sein. Eine Sauerstoffvergiftung der funktionellen Schicht kann weitestgehend verhindert, zumindest aber deutlich verzögert werden.

Die Katalysatorschicht besteht aus einem Metall, das eine hohe Wasserstoffadsorptionsfähigkeit aufweist. Bei einem solchen Metall handelt es sich um ein Platinoid, nämlich Pd oder Pt. Besonders günstig ist dabei die Verwendung des in relativ großen Mengen und im Verhältnis kostengünstig erhältlichen Metalls Pd. Dabei zeichnet sich vor allem Pd dadurch aus, dass es leicht und reaktionsfreudig ist. Die Katalysatorschicht besteht also aus Pd oder Pt, besonders bevorzugt aus Pd. Alternativ wird eine Pd-Pt-Legierung eingesetzt.

Die Deckschicht kann beispielsweise aus Polytetrafluorethylen oder einem modifizierten Polytetrafluorethylen bestehen. Eine dünne gasdurchlässige Polytetrafluorethylen- (PTFE, Teflon) Schicht schützt so einerseits die Katalysatorschicht vor schädlichen Umwelteinflüssen, andererseits kann das Luftgemisch, das den nachzuweisenden Wasserstoff enthält, die Katalysatorschicht problemlos erreichen. Ein Beispiel für ein modifiziertes Polytetrafluorethylen ist sulfoniertes Polytetrafluorethylen (Nafion). Der Sensor weist somit eine deutlich verbesserte Stabilität gegen H₂S auf.

Im Sinne einer einfachen Ausführungsform ist es bei einem solchen Gassensor von Vorteil, dass die Substratschicht aus einem Material ausgewählt aus der Gruppe enthaltend Glas, Quarzglas, Polycarbonat, Saphir, Si besteht. Beispielsweise ist die Substratschicht aus Floatglas hergestellt.

Eine besonders günstige Ausführungsvariante des erfindungsgemäßen Gassensors besteht insofern aus einer Substratschicht aus Glas, auf der eine Mg/Ti-Schicht als funktionelle Schicht gefolgt von einer Ti-Schicht als Zwischenschicht, einer Pd-Schicht als Katalysatorschicht und einer PTFE-Deckschicht aufgebracht sind.

Die Mg/Ti-Schicht hat den Vorteil, dass sie ein besseres Schaltverhalten und eine bessere Stabilität ermöglicht. Sensorsysteme mit Mg/Ti als funktionale Schicht weisen eine größere Änderung der optischen Reflektivität beim Übergang vom metallischen in den Hydridzustand und schnellere Ansprechzeiten (<250ms) auf, sowie eine hohe Stabilität in Bezug auf das gleichbleibende Schaltungsverhalten unter Wasserstoffeinwirkung sowie eine hohe Stabilität in Bezug auf die Struktur der Schichten und der Schichtenabfolge im Messzustand.

Bei einem Übergang zwischen dem metallischen und dem Hydridzustand ändert sich der elektrische Widerstand der funktionellen Schicht. Es ist daher günstig, wenn der Gassensor Elektroden aufweist. Diese können unterhalb der funktionellen Mg/Ti-Schicht ausgebildet sein. Mit ihrer Hilfe sind die Widerstandsänderungen der Mg/Ti-Schicht messbar. Dabei kann es sich beispielsweise um zwei schmale Goldstreifen handeln, die auf das Substrat aufgedampft sind. Die Goldstreifen werden dann durch die Mg/Ti-Schicht bedeckt, so dass sie zwischen dem Substrat und der funktionellen Schicht angeordnet sind.

Daneben ändert sich bei einem Übergang vom metallischen Zustand in den Hydridzustand auch die Transmissivität (optische Transmission) der funktionellen Mg/Ti-Schicht. Eine optische Auswertung des jeweiligen Zustandes kann im einfachsten Fall visuell erfolgen. Vorteilhaft ist aber, wenn der Gassensor wenigstens einen Photodetektor aufweist. Dabei kann es sich beispielsweise um einen Photohalbleiter, etwa einen Photowiderstand, eine Photodiode oder einen Phototransistor, oder um einen Photomultiplier handeln. Der Gassensor ist dabei so gestaltet, dass eine künstliche Lichtquelle auf den Sensor einstrahlt. Der Photodetektor kann dann entweder auf der der Lichtquelle gegenüberliegenden Seite der Sensorschichten angeordnet sein und messen, welcher Lichtanteil die Sensorschichten durchdringt. Alternativ kann er auch in einem bestimmten Winkel zur Lichtquelle auf derselben Seite des Sensors angeordnet sein und messen, wie hoch der reflektierte Anteil des Lichtes ist.

Die Erfindung sieht weiterhin die Verwendung eines erfindungsgemäßen Gassensors in einem mobilen Wasserstoffdetektor vor. Dabei ist es günstig, wenn der Wasserstoffdetektor eine Sensoreinheit, einen Kalibriereinheit, eine Verstärkungseinheit und eine Anzeigeeinheit aufweist, wobei der Gassensor als Sensoreinheit verwendet wird.

Darüber hinaus sieht die Erfindung einen mobilen Wasserstoffdetektor mit einer Sensoreinheit, einem Kalibriereinheit, einer Verstärkungseinheit und einer Anzeigeeinheit vor, wobei die Sensoreinheit ein erfindungsgemäßer Gassensor ist.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1a, 1b: Schematischer Schichtaufbau eines erfindungsgemäßen Gassensors
- Fig. 2a: Schaltverhalten eines erfindungsgemäßen Gassensors bei einer maximalen Wasserstoffkonzentration von 0,25 % bis 4%
- Fig. 2b: Schaltverhalten eines erfindungsgemäßen Gassensors bei Wasserstoffkonzentrationen von 0,01 % bis 0,05 %
- Fig. 3: Spannungsdifferenz am Photodetektor des Gassensors im Verhältnis zur Wasserstoffkonzentration
- Fig. 4a, b, c: Langzeitbelastungstest eines erfindungsgemäßen Gassensors im Vergleich zu herkömmlichen Gassensoren
- Fig. 5a, b: Ansprechzeit eines erfindungsgemäßen Gassensors

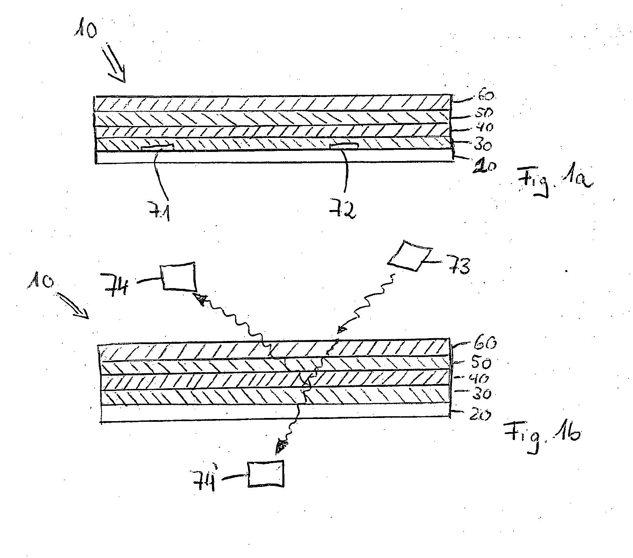

In den Figuren 1a und 1b erkennt man jeweils den schematischen Aufbau eines Gassensors 10 zur Detektion von Wasserstoff. Auf einer Substratschicht 20 ist eine funktionelle Schicht 30 aufgebracht. Diese ist bedeckt von einer Zwischenschicht 40, über der wiederum eine Katalysatorschicht 50 angeordnet ist. Über der Katalysatorschicht 50 ist eine Deckschicht 60 angeordnet.

In Fig. 1a erkennt man außerdem, dass zwischen der Substratschicht 20 und der funktionellen Schicht 30 zwei Elektroden 71, 72 angeordnet sind. Diese Elektroden 71, 72 dienen zur Messung des Widerstandes der funktionellen Schicht 30. Der in Fig. 1 a dargestellte Gassensor 10 ist daher elektrisch auswertbar. Gleichzeitig ist der Gassensor 10 auch optisch auswertbar. Strahlt das Tageslicht oder das umgebende Raumlicht nämlich auf den Gassensor 10, so wird es je nach Zustand der funktionellen Schicht 30 an dieser in einem mehr oder weniger großen Anteil reflektiert. Der nicht reflektierte Teil des Lichtes dringt durch die funktionelle Schicht 30 hindurch. Ist die funktionelle Schicht 30 im metallischen Zustand, so wird nahezu das komplette einfallende Licht reflektiert. Ist die funktionelle Schicht 30 im Hydridzustand, so wird das Licht nahezu vollständig transmittiert. Der Benutzer des Gassensors 10 kann insofern mit bloßem Auge erkennen, ob Wasserstoff in einem Gasgemisch vorliegt, in dem er feststellt, ob das einfallende Licht reflektiert wird oder nicht.

In Fig. 1b ist oberhalb des Gassensors 10 eine Lichtquelle 73 angeordnet, die auf den Gassensor 10 strahlt. Ein Photodetektor 74, 74' ist entweder oberhalb oder unterhalb des Gassensors 10 angeordnet. Der oberhalb des Gassensors 10 angeordnete Photodetektor 74 misst den Anteil des an der funktionellen Schicht 30 reflektierten Lichtes. Der unterhalb des Gassensors 10 angeordnete Photodetektor 74' misst den Anteil des durch die funktionelle Schicht 30 transmittierten Lichtes. Der in Fig. 1b dargestellte Gassensor 10 ist daher optisch auswertbar.

In einem weiteren (nicht dargestellten) Ausführungsbeispiel weist der in Fig. 1 a gezeigte Gassensor 10 zusätzlich zu den Elektroden 71, 72 auch eine Lichtquelle 73 und einen oder mehrere Photodetektoren 74, 74' auf. In einer ersten Variante weist dieser Gassensor 10 zwei Photodetektoren 74, 74' auf, von denen einer oberhalb und einer unterhalb des Gassensors 10 angeordnet ist. Dieser Gassensor 10 erfasst sowohl das reflektierte als auch das transmittierte Licht. In einer zweiten Variante ist der Gassensor 10 nur mit einem Photodetektor 74, 74' ausgestattet, der entweder oberhalb oder unterhalb des Gassensors 10 angeordnet ist. In diesem Fall wird entweder das reflektierte oder das transmittierte Licht gemessen. Ein solcher Gassensor 10 ist sowohl elektrisch als auch optisch auswertbar.

Die Substratschicht 20 des dargestellten Gassensors 10 ist aus Glas. Die funktionelle Schicht 30 ist eine Mg/Ti-Legierung. Die Zwischenschicht 40 besteht aus Ti. Die Katalysatorschicht 50 besteht aus Pd. Die Deckschicht 60 ist aus Polytetrafluorethylen (Teflon).

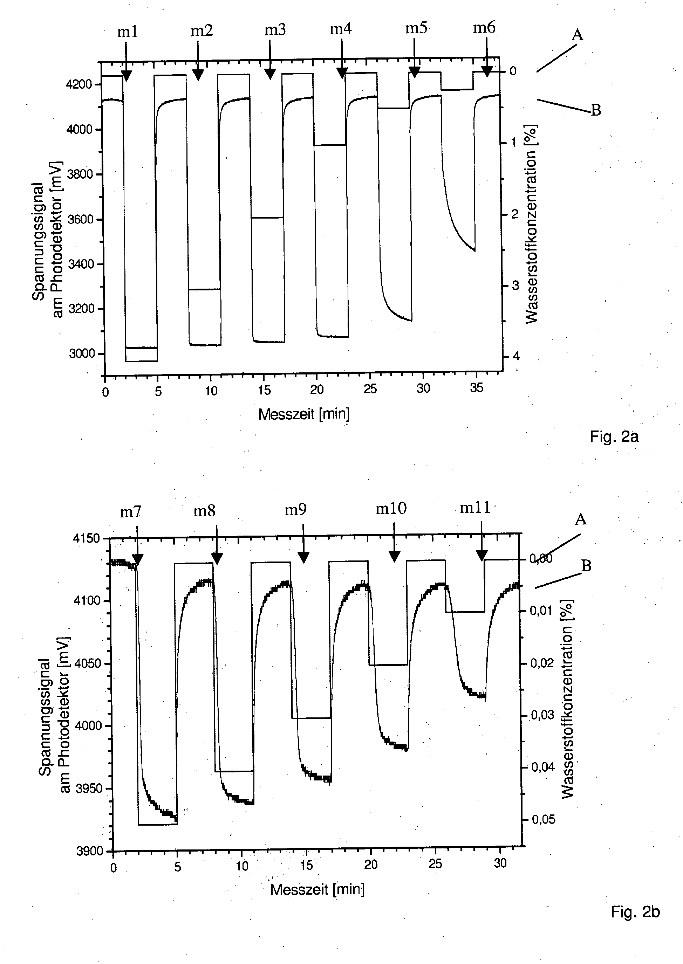

In den Fig. 2a und 2b erkennt man das Reaktionsverhalten des Sensorsystems 10 bei Änderung der Wasserstoffkonzentration in einem Luftgemisch, dem der Gassensor 10 ausgesetzt ist. Das Signal am Photodetektor 74 wird in mV ausgelesen und entspricht der Reflektion der funktionalen Schicht 30. Der Gassensor 10 wird dabei abwechselnd für drei Minuten einem Wasserstoff-Argon-Gemisch und synthetischer Luft ausgesetzt. Fig. 2a zeigt dabei eine Messreihe, die mit einer Nullphase beginnt, dabei wird der Gassensor 10 synthetischer Luft ausgesetzt. Die erste Nullphase wird gefolgt von insgesamt sechs Messphasen m1, m2, m3, m4, m5, m6. Diese dauern jeweils etwa drei Minuten, ebenso wie die zwischen den Messphasen liegenden Nullphasen. In der ersten Messphase m1 wird ein Gasgemisch mit einer Wasserstoffkonzentration von 4 % angelegt, in der zweiten Messphase m2 ein Gasgemisch mit einer Wasserstoffkonzentration von 3 %, in der dritten Messphase m3 ein Gasgemisch mit einer Wasserstoffkonzentration von 2 %, in der vierten Messphase m4 ein Gasgemisch mit einer Wasserstoffkonzentration von 1 %, in der fünften Messphase m5 ein Gasgemisch mit einer Wasserstoffkonzentration von 0,5 % und in der sechsten Messphase ein Gasgemisch mit einer Wasserstoffkonzentration von 0,25 %. Zwischen den Messphasen m1, m2, m3, m4, m5, m6 wird der Gassensor 10 jeweils in einer Nullphase wie bereits oben beschrieben synthetischer Luft ohne Wasserstoff ausgesetzt. Bei der synthetischen Luft handelt es sich um eine auch als Spülgas bezeichnete Mischung von ca. 20 % Sauerstoff in Stickstoff. Das Diagramm in Fig. 2a zeigt auf der Abszisse den Zeitverlauf in Minuten, auf der linken Ordinate ist die vom Transistor gemessene Spannung in mV ablesbar, auf der rechten Ordinate die vorliegende Wasserstoffkonzentration in Prozent. Der Verlauf der Mess- und Nullphasen kann anhand der Kurve A, die die Wasserstoffkonzentration darstellt, erkannt werden. Kurve B zeigt die während der Mess- und Nullphasen auftretende Spannungsänderung am Photodetektor 74. Man erkennt, dass nahezu unmittelbar mit Änderung der Wasserstoffkonzentration auch eine Spannungsänderung einhergeht. Der Gassensor 10 reagiert in allen Messphasen m1, m2, m3, m4, m5, m6 sofort auf den einwirkenden Wasserstoff und hat in < 5 Sekunden 90 % des Sättigungsniveaus erreicht. Auch die Desorption des Wasserstoffs während der Nullphase am Ende der jeweiligen Messphase m1, m2, m3, m4, m5, m6 erfolgt in < 5 Sekunden.

Man erkennt, dass sich der Gassensor 10 in einem Bereich zwischen 0,25 % und 4 % Wasserstoffkonzentration durch schnelle und zuverlässige Anzeige des Wasserstoffs auszeichnet.

Gleiches gilt für einen Bereich zwischen 0,01 und 0,05 % Wasserstoffkonzentration, wie man in Fig. 2b erkennt. Dort sind insgesamt fünf Messphasen m7, m8, m9, m10, m11 dargestellt, die jeweils drei Minuten dauern und wie im Beispiel, dargestellt in Fig. 2A, durch ebenfalls drei Minuten andauernde Nullphasen, in denen der Gassensor 10 synthetischer Luft ausgesetzt ist, unterbrochen werden. In der ersten Messphase m7 wird der Gassensor 10 einem Gasgemisch mit 0,05 % Wasserstoffkonzentration ausgesetzt, in der zweiten Messphase m8 einem Gasgemisch mit 0,04% Wasserstoffkonzentration, in der dritten Messphase m9 einem Gasgemisch mit 0,03 % Wasserstoffkonzentration, in der vierten Messphase m10 einem Gasgemisch mit 0,02 % Wasserstoffkonzentration und in der fünften Messphase m11 einem Gasgemisch mit 0,01 % Wasserstoffkonzentration. Auch hier ist deutlich feststellbar, dass der Gassensor 10 in wenigen Sekunden (< 20 s auf ein Gasgemisch mit 0,05 % bis 0,03 % Wasserstoffkonzentration) auch auf geringe Mengen Wasserstoffs reagiert, wobei auch die Desorption in der Nullphase nach dem Ende der jeweiligen Messphase m7, m8, m9, m10, m11 in < 30 Sekunden vonstatten geht.

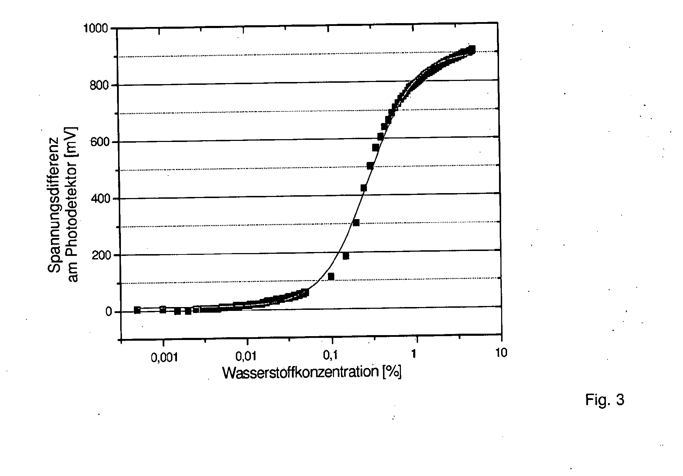

In Fig. 3 erkennt man, dass der Gassensor 10 bei Einwirkung von Wasserstoffgas, insofern also bei einem Übergang der funktionellen Schicht 30 vom metallischen Zustand in den Hydridzustand durch die stattfindende Wasserstoffabsorption mit einer am Photodetektor 74 anfallenden Spannungsänderung zwischen etwa 100 und 900 mV reagiert. Dabei ist auf der Abszisse des Diagramms die am Photodetektor 74 anfallende Spannungsdifferenz in mV und auf der Ordinate des Diagramms die Wasserstoffkonzentration im einwirkenden Gasgemisch dargestellt. Bei einer relativ geringen Wasserstoffkonzentration von etwa 0,01 % ist auch die Spannungsdifferenz mit ungefähr 100 mV gering, bei einer hohen Wasserstoffkonzentration von etwa 4% ist die Spannungsdifferenz mit ungefähr 900 mV ebenfalls hoch. Zwischen diesen Werten steigt die Spannungsdifferenz kontinuierlich mit zunehmender Wasserstoffkonzentration an. Es kann insofern mit Hilfe des Gassensors 10 nicht nur prinzipiell festgestellt werden, dass Wasserstoff in einem Gasgemisch vorhanden ist, sondern es kann auch abgeschätzt werden, ob der Anteil eher gering oder bereits im kritischen Bereich nahe der unteren Explosionsgrenze ist.

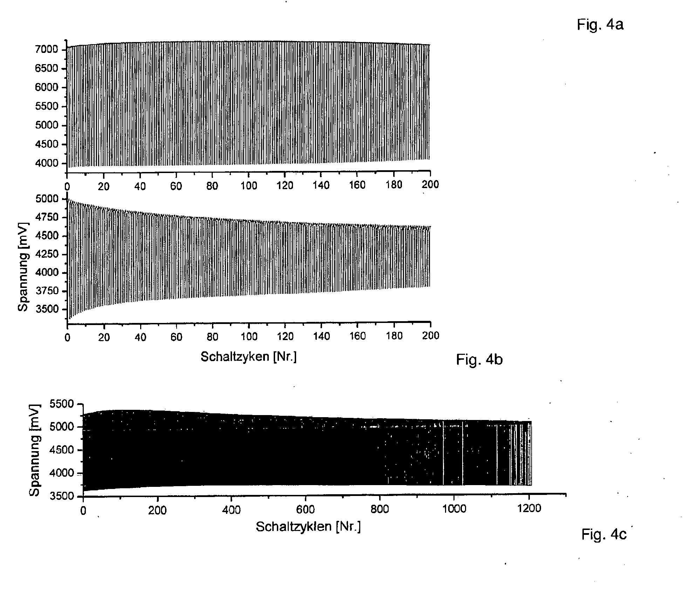

In den Fig. 4a, 4b und 4c erkennt man, dass die Reaktion des Gassensors 10 auch im Laufe einer hohen Anzahl von Schaltzyklen von gleichbleibender Qualität ist. Dabei zeigt Fig. 4b ein Beispiel eines Gassensors aus dem Stand der Technik, Fig. 4a das Langzeitprofil eines erfindungsgemäßen Gassensors 10 über 200 Schaltzyklen und Fig. 4c das Langzeitprofil eines erfindungsgemäßen Gassensors 10 über 1200 Schaltzyklen. In dem in Fig. 4b dargestellten Beispiel aus dem Stand der Technik erkennt man, dass die Amplitude der Spannungsdifferenz, gemessen am Photodetektor 74, bereits nach wenigen Schaltzyklen abnimmt. Im Gegensatz dazu bleibt die Spannungsdifferenz die der erfindungsgemäße Gassensor 10 bei Hinzufügung von Wasserstoff in einem Gasgemisch hervorruft bis zum Ende der Messungen nahezu konstant. In Fig. 4a erkennt man keine Verringerung der Spannungsdifferenz. In Fig. 4c ist nur eine marginale Veränderung erkennbar. Der erfindungsgemäße Gassensor 10 weist insofern eine deutlich längere Stabilität und höhere Zuverlässigkeit auf als ein zum Vergleich gemessener aus dem Stand der Technik bekannter Gassensor.

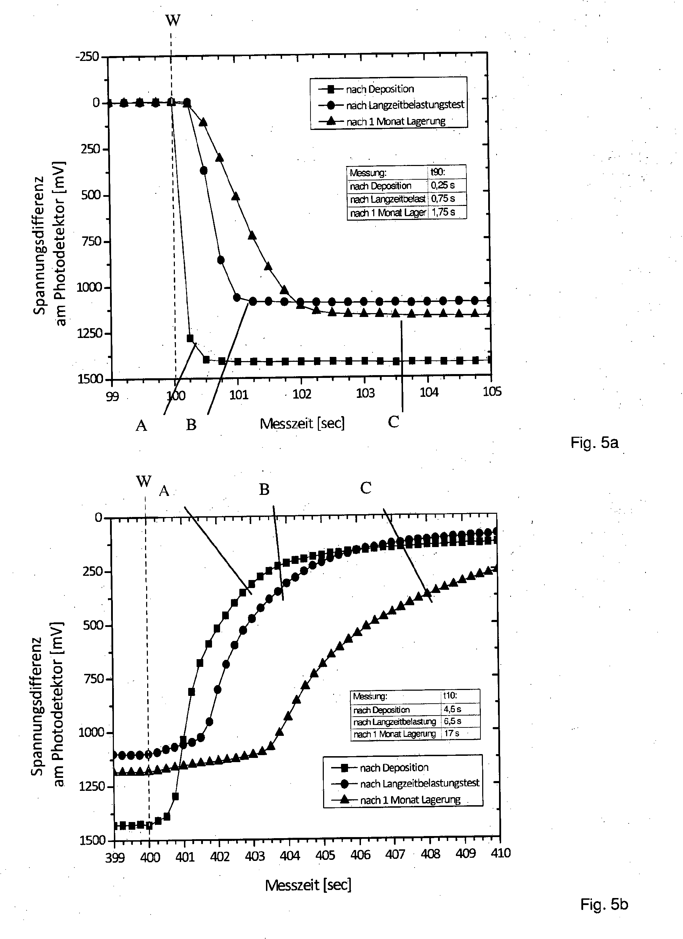

In Fig. 5a ist das Ansprechverhalten eines Gassensors 10 unmittelbar nach Deposition der Schichten des Gassensors 10 (Kurve A mit quadratischen Messpunkten), nach einem Langzeitbelastungstest (Kurve B mit runden Messpunkten) und nach einer einmonatigen Lagerungszeit ohne zwischenzeitliche Schaltung (Kurve C mit dreieckigen Messpunkten) dargestellt. Dabei ist auf der Abszisse die Messzeit in Sekunden dargestellt und auf der Ordinate die Spannung in mV. Die Linie W markiert den Zeitpunkt, ab dem der Gassensor 10 einem Gasgemisch ausgesetzt ist, das eine Konzentration von 4% Wasserstoffgas enthält. Vor diesem Zeitpunkt liegt die funktionelle Schicht 30 im metallischen Zustand vor. Ab diesem Zeitpunkt absorbiert die funktionelle Schicht 30 den durch die Katalysatorschicht 50 dissoziierten Wasserstoff und geht in den Hydridzustand über, was an einer Änderung der Lichtreflektion der Schicht und in der Folge durch eine Spannungsänderung am Photodetektor 74, der das von der Lichtquelle 73 ausgestrahlte Licht detektiert, erkennbar ist.

Man erkennt, dass die größte Spannungsdifferenz und die schnellste Ansprechzeit in Kurve A unmittelbar nach Deposition der Schichten des Gassensors 10 beobachtbar sind. Die Zeit, innerhalb derer der Gassensor 10 eine Wasserstoffsättigung von 90% erreicht - üblicherweise als t₉₀ bezeichnet - beträgt hier 0,25 s. Nach einem Langzeitbelastungstest, welcher etwa 1000 aufeinanderfolgende Schaltzyklen innerhalb von einer Woche umfasst, ist, wie man in Kurve B erkennt, die Spannungsdifferenz, die der Gassensor 10 noch erreicht, etwas geringer als unmittelbar nach Deposition der Schichten. Die Zeit bis zum Erreichen einer 90%igen Sättigung (t₉₀) ist mit 0,75 s etwas länger als in Kurve A. Der Gassensor 10 wird nach dem Langzeitbelastungstest für einen Monat gelagert, das heißt dass er während der Lagerung nicht in Kontakt mit Wasserstoffgas gebracht wird, und anschließend wird ein Schaltvorgang durchgeführt, welcher in Kurve C dargestellt ist. Die Spannungsänderung ist etwas stärker als nach der Langzeitbelastung. Es dauert jedoch deutlich länger bis bei Einwirkung von Wasserstoffgas eine Sättigung von 90% erreicht wird, nämlich 1,75 s. Die Spannungsdifferenz ist insofern bei Kurve A und B, also nach einer Langzeitbelastung bzw. einer einmonatigen Lagerungszeit, geringer, aber immer noch ausreichend, um einen zuverlässigen Nachweis von Wasserstoff in einem Gasgemisch zu ermöglichen.

In Fig. 5b ist das Desorptionsverhalten eines Gassensors 10 unmittelbar nach Deposition der Schichten des Gassensors 10 (Kurve A mit quadratischen Messpunkten), nach einem Langzeitbelastungstest (Kurve B mit runden Messpunkten) und nach einer einmonatigen Lagerungszeit ohne zwischenzeitliche Schaltung (Kurve C mit dreieckigen Messpunkten) dargestellt. Dabei ist auf der Abszisse die Messzeit in Sekunden dargestellt und auf der Ordinate die Spannung am Photodetektor 74 in mV. Die Linie W markiert den Zeitpunkt bis zu dem der Gassensor 10 einem Gasgemisch ausgesetzt ist, das eine 4%ige Konzentration an Wasserstoff enthält. Vor diesem Zeitpunkt liegt die funktionelle Schicht 30 im Hydridzustand vor. Ab diesem Zeitpunkt desorbiert der eingelagerte Wasserstoff aus der funktionellen Schicht 30. In der Folge geht die funktionelle Schicht 30 wieder in den metallischen Zustand über, was wiederum an einer Änderung der Reflektion der Schicht und in der Folge durch eine Spannungsänderung am Photodetektor 74 erkennbar ist.

Auch hier ist in Kurve A, also unmittelbar nach Deposition der den Gassensor 10 bildenden Schichten, die schnellste Reaktion zu beobachten. Die Reaktionszeit, innerhalb derer der Gassensor 10 10% des Endspannungssignals erreicht - üblicherweise als t₁₀ bezeichnet - beträgt hier 4,5 s. Nach einem Langzeitbelastungstest ist die Reaktionszeit mit 6,5 s, wie in Kurve B erkennbar, nur etwas länger als in Kurve A. Am längsten dauert die Desorption nach einem einmonatigen Lagerintervall ohne zwischenzeitliche Schaltung, wie in Kurve C erkennbar. Dabei dauert es 17 s bis die maximale Desorption erreicht ist. Der erste Anstieg der Spannung erfolgt jedoch nur geringfügig langsamer als in den Kurven A und B. Erst gegen Ende des Anstiegs flacht die Kurve C ab, weshalb es zur längeren Reaktionszeit kommt. Es ist jedoch auch hier innerhalb von weniger als 10 s eine eindeutige Reaktion feststellbar.

Auch die detektierte Spannungsdifferenz zwischen dem mit Wasserstoff beladenen Hydridzustand und dem metallischen Zustand ohne eingelagerten Wasserstoff ist in Kurve A am größten. In Kurve B ist die Spannungsdifferenz geringer, d.h. im metallischen Zustand wird nur ein geringeres Spannungsniveau erreicht. In Kurve C ist die Spannungsdifferenz etwa so hoch wie in Kurve B.

Man erkennt insgesamt in den Fig. 5a und 5b, dass das Beste, im Sinne vom schnellsten und deutlichsten Ansprechverhalten eines erfindungsgemäßen Gassensors 10, unmittelbar nach Deposition der den Gassensor 10 bildenden Schichten erreicht wird. Auch nach Langzeitbelastung oder nach einer gewissen Lagerperiode ist der erfindungsgemäße Gassensor 10 aber immer noch in der Lage, ausreichend schnell und zuverlässig das Vorhandensein von Wasserstoff in einem Gasgemisch anzuzeigen.

Die Erfindung ist nicht auf eine der vorbeschriebenen Ausführungsformen beschränkt, sondern in vielfältiger Weise abwandelbar.

In einer ersten Variante ist der Photodetektor 74, 74' ein Phototransistor, in einer zweiten Variante eine Photodiode, in einer dritten Variante ein Photowiderstand und in einer vierten Variante ein Photomultiplier.

Die Substratschicht 20 ist in einer ersten Variante aus Floatglas, in einer zweiten Variante aus einem Kunststoff z.B. Polycarbonat.

Die Zwischenschicht 40 besteht in einer ersten Variante aus Ti. In einer zweiten Variante kann die Zwischenschicht 40 auch aus V oder einem Gemisch aus Ti und V bestehen. Alternativ wird diese Zwischenschicht zusätzlich mit Mg dotiert.

Die Katalysatorschicht 50 wird in einer ersten alternativen Variante aus Pd oder einem Gemisch aus Pd und Pt gebildet.

Die Deckschicht 60 wird alternativ zu Polytetratfluorethylen auch aus einem modifizierten Polytetrafluorethylen bestehen.

Man erkennt, dass es bei einem Gassensor 10 zur Detektion von Wasserstoffgas, wobei der Gassensor 10 wenigstens eine Substratschicht 20, eine funktionelle Schicht 30 und eine Katalysatorschicht 50 aufweist, wobei die funktionelle Schicht 30 zwischen der Substratschicht 20 und der Katalysatorschicht 50 angeordnet ist, von Vorteil ist, wenn zwischen der funktionellen Schicht 30 und der Katalysatorschicht 50 eine Zwischenschicht 40 angeordnet ist und wenn sich eine Deckschicht 60 auf der Katalysatorschicht 50 befindet und dass die funktionelle Schicht 30 eine Magnesiumtitanlegierung ist.

Die Zwischenschicht 40 besteht aus einem hydrierbaren Metall, nämlich aus einem Übergangsmetall, bevorzugt aus Ti oder V, besonders bevorzugt aus Ti. Die Katalysatorschicht 50 besteht aus einem Metall, das eine hohe Wasserstoffabsorptionsfähigkeit aufweist, nämlich aus Pd oder Pt, bevorzugt aus Pd. Weiterhin ist es günstig, wenn die Deckschicht 60 aus Polytetrafluorethylen oder einem modifizierten Polytetrafluorethylen besteht. Die Substratschicht 20 ist aus einem Material ausgewählt aus der Gruppe enthaltend Glas, Quarzglas, Polycarbonat, Saphir, Si.

Man erkennt weiterhin, dass es günstig ist, wenn der Gassensor 10 Elektroden 71, 72 aufweist und wenn der Gassensor 10 wenigstens einen Photodetektor 74, 74' aufweist.

Darüber hinaus stellt man den Vorteil fest, der in der Verwendung eines erfindungsgemäßen Gassensors 10 in einem mobilen Wasserstoffdetektor liegt. Dabei ist es sinnvoll, wenn der Wasserstoffdetektor eine Sensoreinheit, einen Kalibriereinheit, eine Verstärkungseinheit und eine Anzeigeeinheit aufweist, wobei der Gassensor 10 als Sensoreinheit verwendet wird.

Ein weiterer Vorteil der Erfindung liegt insofern in einem mobilen Wasserstoffdetektor mit einer Sensoreinheit, einer Kalibriereinheit, einer Verstärkungseinheit und einer Anzeigeeinheit, wobei die Sensoreinheit ein erfindungsgemäßer Gassensor 10 ist.

### Bezugszeichenliste

- A: Kurve
- B: Kurve
- C: Kurve
- m1, m2, m3, m4, m5, m6, m7,m8, m9, m10, m11: Messphase

- 10: Gassensor
- 20: Substratschicht
- 30: funktionelle Schicht
- 40: Zwischenschicht
- 50: Katalysatorschicht
- 60: Deckschicht

- 71: Elektrode
- 72: Elektrode
- 73: Lichtquelle
- 74: Photodetektor
- 74': Photodetektor

## Patentansprüche

1. Gassensor (10) zur Detektion von Wasserstoffgas, wobei der Gassensor (10) wenigstens eine Substratschicht (20), eine funktionelle Schicht (30) und eine Katalysatorschicht (50) aufweist, wobei die funktionelle Schicht (30) zwischen der Substratschicht (20) und der Katalysatorschicht (50) angeordnet ist, zwischen der funktionellen Schicht (30) und der Katalysatorschicht (50) eine Zwischenschicht (40) und auf der Katalysatorschicht (50) eine Deckschicht (60) angeordnet sind, die Zwischenschicht (40) aus einem hydrierbaren Metall, nämlich einem Übergangsmetall, besteht, **dadurch gekennzeichnet, dass** die Substratschicht (20) aus einem Material ausgewählt aus der Gruppe enthaltend Glas, Quarzglas, Polycarbonat, Saphir, Si besteht und die Katalysatorschicht (50) aus Pd, Pt oder einer Pd-Pt-Legierung besteht und die funktionelle Schicht (30) eine Mg/Ti-Schicht ist.

2. Gassensor entsprechend Anspruch 1, **dadurch gekennzeichnet, dass** die Zwischenschicht (40) aus Ti oder V, bevorzugt aus Ti, besteht.

3. Gassensor nach einem der Ansprüche 1 oder 2, **dadurch gekennzeichnet, dass** die Katalysatorschicht (50) eine hohe Wasserstoffabsorptionsfähigkeit aufweist.

4. Gassensor nach einem der Ansprüche 1 bis 3, **dadurch gekennzeichnet, dass** die Katalysatorschicht (50) aus Pd besteht.

5. Gassensor nach einem der Ansprüche 1 bis 4, **dadurch gekennzeichnet, dass** die Deckschicht (60) aus Polytetrafluorethylen oder einem modifizierten Polytetrafluorethylen besteht.

6. Gassensor nach einem der Ansprüche 1 bis 5, **dadurch gekennzeichnet, dass** der Gassensor (10) Elektroden (71, 72) aufweist.

7. Gassensor nach einem der Ansprüche 1 bis 6, **dadurch gekennzeichnet, dass** der Gassensor (10) wenigstens einen Photodetektor (74, 74') aufweist.

8. Verwendung eines Gassensors (10) nach einem der Ansprüche 1 bis 7 in einem mobilen Wasserstoffdetektor.

9. Verwendung nach Anspruch 8, **dadurch gekennzeichnet, dass** der Wasserstoffdetektor eine Sensoreinheit, eine Kalibriereinheit, eine Verstärkungseinheit und eine Anzeigeeinheit aufweist, wobei der Gassensor (10) als Sensoreinheit verwendet wird.

10. Mobiler Wasserstoffdetektor mit einer Sensoreinheit, einer Kalibriereinheit, einer Verstärkungseinheit und einer Anzeigeeinheit, wobei die Sensoreinheit ein Gassensor (10) nach einem der Ansprüche 1 bis 7 ist.

## Claims

1. Gas sensor (10) for detecting hydrogen gas, with the gas sensor (10) having at least a substrate layer (20), a functional layer (30) and a catalyst layer (50), with the functional layer (30) arranged between the substrate layer (20) and the catalyst layer (50), between the functional layer (30) and the catalyst layer (50) an in-between layer (40) and on the catalyst layer (50) a top layer (60) are arranged, the in-between layer (40) consisting of a hydratable metal, i.e. a transition metal, **characterized to the extent** that the
substrate layer (20) consists of a material selected from the group containing glass, quartz glass, polycarbonate, sapphire, Si and the catalyst layer (50) consisting of Pd, Pt or a Pd-Pt alloy and the functional layer (30) is an Mg/Ti layer.

2. Gas sensor corresponding to Claim 1 **characterized to the extent that** the in-between layer (40) consists of Ti or V, preferably of Ti.

3. Gas sensor in keeping with one of the Claims 1 or 2 **characterized to the extent** that the catalyst layer (50) has a high hydrogen absorption capacity.

4. Gas sensor in keeping with one of the Claims 1 to 3 **characterized to the extent** that the catalyst layer (50) consists of Pd.

5. Gas sensor in keeping with one of the Claims 1 to 4 **characterized to the extent** that the top layer (60) consists of polytetrafluorethylene or a modified polytetrafluorethylene.

6. Gas sensor in keeping with one of the Claims 1 to 5 **characterized to the extent** that the gas sensor (10) has electrodes (71, 72).

7. Gas sensor in keeping with one of the Claims 1 to 6 **characterized to the extent** that the gas sensor (10) at least has a photo detector (74, 74).

8. Use of a gas sensor (10) in keeping with one of the Claims 1 to 7 in a mobile hydrogen detector.

9. Use in keeping with Claim 8 **characterized to the extent** that the hydrogen detector has a sensor unit, a calibrating unit, an amplification unit and a display unit with the gas sensor (10) being used as a sensor unit.

10. Mobile hydrogen detector with a sensor unit, a calibrating unit, an amplification unit and a display unit with the sensor unit being a gas sensor (10) in keeping with one of the Claims 1 to 7.

## Revendications

1. Détecteur de gaz (10) pour la détection d'hydrogène gazeux, constitué d'au moins une couche de substrat (20), une couche fonctionnelle (30) et une couche de catalyseur (50), dont la couche fonctionnelle (30) se situe entre la couche de substrat (20) et la couche de catalyseur (50), dont la couche fonctionnelle (30) et la couche de catalyseur (50) présentent une couche intermédiaire (40) et dont la couche de catalyseur (50) est recouverte d'une couche de protection (60), la couche intermédiaire (40) étant composée d'un métal hydrurable, à savoir un métal de transition, **caractérisé en ce que** la couche de substrat (20) est composée d'un matériau issu du groupe comprenant le verre, le verre de quartz, le polycarbonate, le saphir, le silicium et la couche de catalyseur (50) est élaborée avec du palladium, du platine ou un alliage des deux, tandis que la couche fonctionnelle (30) forme une couche de magnésium/titane.

2. Détecteur de gaz selon la revendication 1, **caractérisé en ce que** la couche intermédiaire (40) est composée de titane ou de vanadium, de préférence de titane.

3. Détecteur de gaz selon la revendication 1 ou 2, **caractérisé en ce que** la couche de catalyseur (50) présente une capacité d'absorption d'hydrogène élevée.

4. Détecteur de gaz selon l'une des revendications 1 à 3, **caractérisé en ce que** la couche de catalyseur (50) est composée de palladium.

5. Détecteur de gaz selon l'une des revendications 1 à 4, **caractérisé en ce que** la couche de protection (60) est composée de polytétrafluoréthylène ou d'un polytétrafluoréthylène modifié.

6. Détecteur de gaz selon l'une des revendications 1 à 5, **caractérisé en ce que** le détecteur de gaz (10) est doté d'électrodes (71, 72).

7. Détecteur de gaz selon l'une des revendications 1 à 6, **caractérisé en ce que** le détecteur de gaz (10) est doté d'au moins un détecteur photosensible (74, 74').

8. Utilisation d'un détecteur de gaz (10) selon l'une des revendications 1 à 7 dans un détecteur d'hydrogène mobile.

9. Utilisation selon la revendication 8, **caractérisée en ce que** le détecteur d'hydrogène comporte une unité de détection, une unité de calibrage, une unité d'amplification ainsi qu'une unité d'affichage, et dans lequel le détecteur de gaz (10) est employé comme unité de détection.

10. Détecteur d'hydrogène mobile doté d'une unité de détection, une unité de calibrage, une unité d'amplification ainsi qu'une unité d'affichage, et dans lequel l'unité de détection est un détecteur de gaz (10) selon les revendications 1 à 7.
